# EUROPÄISCHE PATENTANMELDUNG

(11) **EP 0 577 559 A2**
(43) Veröffentlichungstag der Anmeldung: **05.01.1994**
(21) Anmeldenummer: 93810453.6
(22) Anmeldetag: 24.06.1993
(51) Int. Cl.: C07D 251/20, C07D 251/22

(54) **Verfahren zur Herstellung von Mono- und Diaryltriazinen**

(30) Priorität: 02.07.1992 CH 2086/92
(71) Anmelder: CIBA-GEIGY AG, CH-4002 Basel (CH)
(72) Erfinder: Henneberger, Helmut, Dr., D-7888 Rheinfelden (DE); Wagner, Markus, CH-4414 Füllinsdorf (CH)

(57) **Zusammenfassung**

Es wird ist ein Verfahren beschrieben zur selektiven Herstellung von 2,4-Dichlor-6-aryl-1,3,5-triazinen oder 2-Chlor-4,6-diaryl-1,3,5-triazinen, dadurch gekennzeichnet, daß man eine Verbindung der Formel II 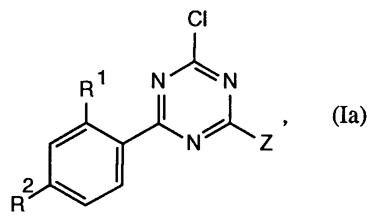
worin X für ein Brom- oder ein Chloratom steht und R¹ und R² die oben angegebenen Bedeutungen haben, in Tetrahydrofuran metallischem Magnesium zur entsprechenden Grignard-Verbindung reagieren läßt und die erhaltene Lösung mit Cyanursäurechlorid der Formel III 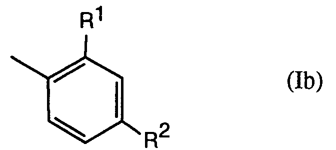
umsetzt, wobei im Fall von Monoaryltriazinen auf 1 Mol Cyanursäurechlorid mindestens 1,05 Mol Grignard-Verbindung und im Fall von Diaryltriazinen auf 1 Mol Cyanursäurechlorid mindestens 2,1 Mol der Grignard-Verbindung eingesetzt werden. Die Verbindungen der Formel I lassen sich vorteilhaft zur Herstellung von 2-(o-Hydroxyphenyl)-4,6-diaryl-s-triazinen einsetzen, die als Lichtstabilisatoren für organisches Material bekannt sind.

## Beschreibung

Die Erfindung betrifft ein Verfahren zur selektiven Herstellung von 2,4-Dichlor-6-aryl- oder 2-Chlor-4,6-diaryl- 1,3,5-triazinen aus Cyanursäurechlorid und Arylmagnesiumhalogeniden.

Dreifach arylsubstituierte Triazine sind beispielsweise durch Umsetzung von Benzamidin mit Resorcinderivaten unter Aufbau des Triazinringes zuganglich (US-A-3 268 474). Andere Synthesen gehen von Cyanursäurehalogeniden aus, die mit Phenylderivaten nach Friedel-Crafts oder nach Grignard umgesetzt werden (siehe z.B. US-A-3 244 708). Einen guten Überblick über die verschiedenen Synthesemethoden bieten beispielsweise E. M. Smolin und L. Rapoport, s-Triazines and Derivatives, Interscience Publishers Inc., New York 1959, sowie US-A-3 268 474 und US-A-3 118 887.

Die Substitution von 1 oder 2 Chloratomen des Cyanursäurechlorids durch Phenyl mit Hilfe einer Grignard-Reaktion wurde bereits beschrieben durch A. Ostrogovich, Chemiker-Zeitung Nr.78, 738 (1912), und Hirt et al., Helv. Chim. Acta 178-179, 1365 (1950).

US-A-3 268 528 und Bader et al., J. Org. Chem. 30, 702 (1965), beschreiben die Synthese von 2-Chlor-4,6-dialkyl-1,3,5-triazin mit Hilfe von Grignard-Reaktionen, wobei bei -15°C gearbeitet wird. Bader et al. untersuchen den Einfluß verschiedener Lösungsmittel auf die Ausbeute und die Art der erhaltenen Nebenprodukte; sie beobachten bei Einsatz von Tetrahydrofuran eine beschleunigte Substitution von Chlor und einen erhöhten Anteil an Nebenreaktionen.

US-A-4 092 466 gibt eine Methode zur Herstellung von 2-Chlor-4,6-diphenyl-1,3,5-triazin an, worin zunächst in üblicher Weise aus Brombenzol und Magnesium in Diethylether die Grignard-Lösung hergestellt wird; diese wird anschließend mit einer Lösung von Cyanursäurechlorid in Benzol vereinigt und das Reaktionsgemisch bei 4 bis 15°C zum Rohprodukt umgesetzt.

Gemeinsames Merkmal der bekannten Verfahren zur selektiven Substitution von 1 oder 2 Chloratomen des Cyanursäurechlorid ist die Bildung eines hohen Anteils unerwünschter Nebenprodukte.

Es wurde nun gefunden, daß Cyanursäurechlorid sich mit in Tetrahydrofuran gelösten Arylmagnesiumhalogeniden überraschenderweise in hoher Ausbeute selektiv zu 2,4-Dichlor-6-aryl-1,3,5-triazinen oder selektiv zu 2-Chlor-4,6-diaryl-1,3,5-triazinen umsetzen läßt Gegenstand der Erfindung ist daher ein Verfahren zur Herstellung einer Einzelverbindung der Formel Ia, 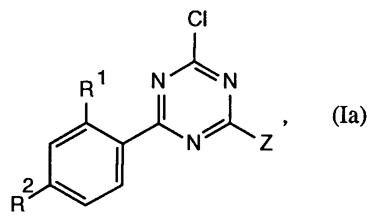
worin R¹ und R² unabhängig voneinander Wasserstoff oder C₁-C₄-Alkyl darstellen, und Z ein Rest der Formel Ib 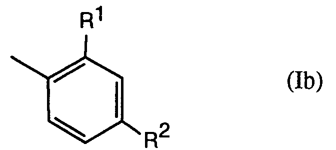
oder -Cl ist,
dadurch gekennzeichnet, daß man eine Verbindung der Formel II 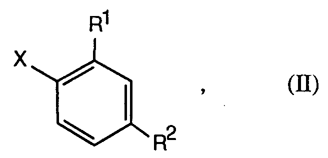
worin X für ein Brom- oder ein Chloratom steht und R¹ und R² die oben angegebenen Bedeutungen haben, in Tetrahydrofuran mit metallischem Magnesium zur entsprechenden Grignard-Verbindung reagieren laßt und die erhaltene Lösung mit Cyanursaurechlorid der Formel III 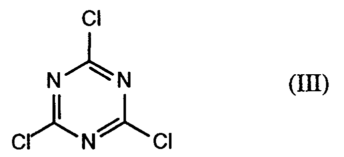
umsetzt, wobei auf 1 Mol Cyanursäurechlorid im Fall, daß Z -Cl ist, mindestens 1,05 Mol der Grignard-Verbindung eingesetzt werden, und im Fall, daß Z ein Rest der Formel Ib ist, mindestens 2,1 Mol der Grignard-Verbindung eingesetzt werden.

Ein bevorzugter Gegenstand der Erfindung ist ein Verfahren zur Herstellung von 2-Chlor-4,6-diaryl-1,3,5-triazinen der Formel I, 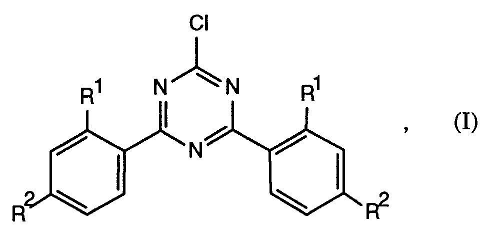
worin R¹ und R² unabhängig voneinander Wasserstoff oder C₁-C₄-Alkyl darstellen, dadurch gekennzeichnet, daß man eine Verbindung der Formel II 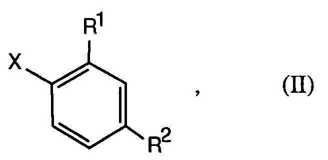
worin X für ein Brom- oder ein Chloratom steht und R¹ und R² die oben angegebenen Bedeutungen haben, in Tetrahydrofuran mit metallischem Magnesium zur entsprechenden Grignard-Verbindung reagieren läßt und die erhaltene Lösung mit Cyanursäurechlorid der Formel III 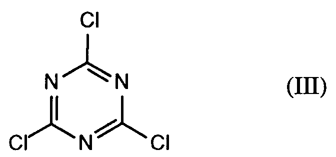
umsetzt, wobei auf 1 Mol Cyanursäurechlorid mindestens 2,1 Mol der Grignard-Verbindung eingesetzt werden.

Das Umsetzungsprodukt der Verbindung der Formel II mit metallischem Magnesium ist dem Fachmann als Grignard-Verbindung bekannt. Bei der Herstellung der Grignard-Verbindung im erfindungsgemäßen Verfahren ist neben Tetrahydrofuran (THF) kein weiteres Lösungsmittel anwesend.

Ein Verfahren, worin auch bei der Umsetzung mit dem Cyanursäurechlorid neben Tetrahydrofuran (THF) kein weiteres Lösungsmittel eingesetzt wird, stellt eine bevorzugte Ausführungsform der Erfindung dar.
In diesem Fall wird Cyanursäurechlorid zweckmäßig in Tetrahydrofuran gelöst und diese Lösung anschließend mit der Lösung der Grignard-Verbindung vereinigt.

Ebenfalls möglich ist es, bei der Umsetzung mit dem Cyanursäurechlorid neben Tetrahydrofuran ein zusätzliches Lösungsmittel einzusetzen. Dafür eignen sich beispielsweise acyclische oder andere cyclische Ether oder aromatische oder aliphatische Kohlenwasserstoffe; bevorzugt sind Dioxan, Toluol, Xylol, Hexan, Cyclohexan, Petrolether oder Mischungen dieser Lösungsmittel, vor allem Toluol.
In diesem Fall wird Cyanursäurechlorid zweckmäßig als Lösung in dem zusätzlichen Lösungsmittel vorgelegt und dazu die Lösung des Grignard-Reagens gegeben.

Ein Verfahren, worin Cyanursäurechlorid als Lösung in Tetrahydrofuran, Toluol oder einer Mischung aus Toluol und Tetrahydrofuran vorgelegt und dazu die Grignard-Verbindung als Lösung in Tetrahydrofuran zugegeben wird, stellt ebenfalls eine bevorzugte Ausführungsform der Erfindung dar.

Die Konzentration des vorgelegten Cyanursäurechlorid beträgt vorzugsweise 1 bis 3 Mol pro Liter Lösungsmittel.

Die Dauer der Reaktion hängt von mehreren Parametern ab, beispielsweise der Art des Substituenten Z (Produkt mono- oder diaryliert), der Art der Substituenten der Grignard-Verbindung X, R¹ und R², sowie der gewählten Temperatur. Im Allgemeinen beträgt die gesamte Reaktionsdauer einschließlich der für das Zudosieren der Grignard-Lösung benötigten Zeit 1 bis 24 Stunden, wobei die Lösung der Grignard-Verbindung innerhalb von 0,5 bis 3 Stunden zudosiert wird. Vorzugsweise beträgt die Reaktionsdauer bei Umsetzung von Grignard-Verbindungen aus Edukten der Formel II mit R¹ = H 1 bis 6, vor allem 2 bis 5 Stunden.

Vorzugsweise wird die Reaktionsmischung während des Vermischens von Grignard-Verbindung und Cyanursäurechlorid sowie während der gesamten Dauer der Umsetzung gerührt und im Temperaturbereich von -5°C bis +45°C, beispielsweise im Temperaturbereich von -5°C bis +30°C, besonders im Temperaturbereich von 0°C bis 25°C, vor allem im Temperaturbereich von 5°C bis 20°C gehalten.

Die Grignard-Verbindung wird zweckmäßig in 10 bis 100 % stöchiometrischem Überschuß eingesetzt, so daß auf ein Mol Cyanursäurechlorid im Fall, daß Z -Cl ist, zweckmäßig 1,1 bis 2 Mole der Grignard-Verbindung eingesetzt werden, und im Fall, daß Z ein Rest der Formel Ib ist, zweckmäßig 2,2 bis 4 Mole der Grignard-Verbindung kommen. Im Allgemeinen beträgt der Überschuß 10 bis 75 %; bevorzugt beträgt er 25 bis 50 %, d.h. auf ein Mol Cyanursäurechlorid werden im Fall, daß Z -Cl ist, bevorzugt 1,25 bis 1,5 Mol der Grignard-Verbindung eingesetzt, und im Fall, daß Z ein Rest der Formel Ib ist, bevorzugt 2,5 bis 3 Mole des Umsetzungsproduktes der Verbindung der Formel II mit Magnesium eingesetzt.

Zur Herstellung der Grignard-Verbindung kann ein einheitliches Edukt der Formel II mit X = Br oder X = Cl eingesetzt werden; es ist jedoch auch möglich, Gemische aus Brom- und Chlorverbindungen einzusetzen.

Der Umsetzungsgrad wird zweckmäßig während der Reaktion durch die Analyse von Proben der Reaktionsmischung verfolgt. Die Analyse kann beispielsweise mit Hilfe der Dünnschicht-Chromatographie erfolgen.

Die Aufarbeitung der Reaktionsmischung kann in bekannter Weise erfolgen, beispielsweise durch Verdünnen mit einem unpolaren organischen Lösungsmittel, Hydrolysieren der überschüssigen Grignard-Verbindung, Abtrennen, Waschen und Trocknen der organischen Phase und Entfernen des Lösungsmittels. Zum Verdünnen eignen sich beispielsweise aliphatische oder aromatische Kohlenwasserstoffe wie z.B. Petrolether, Toluol oder Xylol; besonders geeignet ist Toluol. Die Hydrolyse der nicht umgesetzten Grignard-Verbindung erfolgt zweckmäßig durch Zugabe einer ausreichenden Menge verdünnter Mineralsäuren wie z.B. 0,1 bis 5 molarer wässriger HCl. Zur Hydrolyse wird vorteilhaft ein Überschuß an Säure eingesetzt, beispielsweise auf 1 Mol nicht umgesetzter Grignard-Verbindung 2 bis 5 Mol wässrige HCl.

Das Verfahrensprodukt, die Verbindung der Formel Ia oder die Verbindung der Formel I, wird in hoher Reinheit als Einzelverbindung erhalten, so daß eine destillative Aufreinigung zweckmäßig entfällt. Eine gegebenenfalls gewünschte weitere Aufreinigung kann beispielsweise durch Umkristallisieren erfolgen. Die dafür in Frage kommenden Lösungsmittel sind dem Fachmann bekannt; beispielsweise können polare oder unpolare organische Lösungsmittel wie aliphatische oder aromatische Kohlenwasserstoffe, Alkohole, Amide oder Ether verwendet werden. Vorteilhaft einsetzbare Alkohole sind z.B. C₃-C₅-Alkohole wie 2-Butanol.

Die im erfindungsgemaßen Verfahren eingesetzte Grignard-Verbindung ist in Tetrahydrofuran ohne Zusatz weiterer Lösungsmittel gelöst Die Herstellung der Grignard-Verbindung in Tetrahydrofuran kann auf bekannte Weise erfolgen. Beispielsweise wird metallisches Magnesium in geeigneter Form, z.B. als Späne, Pulver oder Streifen, zunächst aktiviert; dies kann durch kurzes Erhitzen in THF geschehen, möglich ist auch der Zusatz einer katalytischen Menge eines aktivierenden Stoffes, z.B. Iod. Anschließend wird die Verbindung der Formel II in ungefähr äquivalenter Menge oder in bis ca. 10 % molarem Überschuß zugesetzt, beispielsweise als Lösung in THF. Im Fall des Einsatzes von Verbindungen der Formel II, worin X für ein Chloratom steht, kann zur Aktivierung vorteilhaft eine zusätzliche geringe Menge der entsprechenden Bromverbindung, z.B. 1 bis 10 Gew.-% bezogen auf die Chlorverbindung, zugesetzt werden. Die Mischung erwärmt sich anschließend infolge der exothermen Reaktion oder wird auf eine Temperatur im Bereich von ca. 25°C bis zum Siedepunkt des THF, vorzugsweise auf Rückflußtemperatur, erhitzt. Die Reaktion wird zweckmäßig fortgesetzt, bis das eingesetzte Magnesium vollständig gelöst ist. Die Menge an Lösungsmittel wird bevorzugt so bemessen, daß auf 1 Mol der Grignard-Verbindung 3 bis 3,5 Mole THF kommen.

Von besonderem technischem Interesse zur Verwendung im erfindungsgemäßen Verfahren sind Verbindungen der Formel II, worin X für ein Chloratom steht.

R¹ und R² als C₁-C₄-Alkyl bedeuten Methyl, Ethyl, 1-Propyl (n-Propyl), 2-Propyl (Isopropyl), 1-Butyl (n-Butyl), 2-Butyl (sec.-Butyl), 2-Methylpropyl (Isobutyl) oder 1,1-Dimethylethyl (tert.-Butyl); besonders Methyl oder tert.-Butyl.

Vorzugsweise hat R¹ die Bedeutung Wasserstoff oder Methyl und R² bedeutet Wasserstoff oder C₁-C₄-Alkyl. Besonders bevorzugt bedeuten R¹ und R² unabhängig voneinander Wasserstoff oder Methyl. In einer besonders bevorzugten Ausführungsform der Erfindung handelt es sich bei der Verbindung der Formel II um Chlorbenzol, Brombenzol, 4-Chlortoluol, 4-Brom-toluol, 2,4-Dimethyl-brombenzol oder 2,4-Dimethyl-chlorbenzol.

Ein besonders bevorzugter Gegenstand der Erfindung ist daher ein Verfahren, worin Cyanursäurechlorid als Lösung in Tetrahydrofuran, Toluol oder einer Mischung aus Toluol und Tetrahydrofuran vorgelegt und auf 1 Mol Cyanursäurechlorid unter Rühren und Kühlen eine Lösung von 2,5 bis 3 Molen des Umsetzungsproduktes von Chlorbenzol, Brombenzol, 4-Chlor-toluol, 4-Brom-toluol, 2,4-Dimethyl-brombenzol oder 2,4-Dimethyl-chlorbenzol mit Magnesium in Tetrahydrofuran zugegeben wird.

Die Verbindungen der Formel Ia oder I lassen sich vorteilhaft zur Herstellung von 2-(o-Hydroxyphenyl)-4,6-diaryl-s-triazinen einsetzen, die als Lichtstabilisatoren für organisches Material bekannt sind. Beispiele für diese Verwendung von Verbindungen der Formel Ia oder I finden sich beispielsweise in US-A-3 244 708.

Die nachfolgenden Beispiele illustrieren das erfindungsgemäße Verfahren weiter. Alle Angaben in Teilen oder Prozenten beziehen sich auf das Gewicht, soweit nicht anders angegeben.

### Beispiel 1: Herstellung von 2-Chlor-4,6-diphenyl-1,3,5-triazin

24,30 g (1,0 Mol) mit Iod aktivierte Magnesiumspäne in 40 ml wasserfreiem Tetrahydrofuran (THF) werden kurzzeitig bis auf ca. 60°C aufgeheizt. Darauf werden 157,0 g (1,0 Mol) Brombenzol und 220 ml THF innerhalb von 30 Min. zugetropft und anschließend für 2 Stunden auf Rückflußtemperatur gehalten. Nach Abkühlen wird die Grignard-Lösung innerhalb von 2 Stunden zu einer Mischung von 61,4 g (0,33 Mol) Cyanursäurechlorid und 215 ml THF gegeben, wobei die Temperatur der Mischung im Bereich von 0 bis 20°C gehalten wird. Nach beendeter Zugabe wird die Mischung für eine weitere Stunde bei 20°C gerührt; zu diesem Zeitpunkt ist im Dünnschicht-Chromatogramm (Laufmittel Toluol:Hexan = 1:1) kein Monoaryltriazin mehr zu erkennen.
Anschließend wird die Mischung mit 300 ml Toluol verdünnt, die erhaltene Suspension auf 316 g 12 % wässrige HCl gegossen, die organische Phase abgetrennt, mit insgesamt 400 g Wasser gewaschen und eingeengt. Das erhaltene Material (Schmelzpunkt 128-134°C) enthält 92 % der Titelverbindung (Ausbeute 99,5 % d.Th.). Nach Umkristallisieren aus 2-Butanol erhält man die Titelverbindung vom Schmelzpunkt 136-139°C.

| | |
|---|---|
| Analyse C₁₅H₁₀Cl N₃: | ber. 67,30 % C; 3,76 % H; 15,70 % N; 13,24 % Cl; |
| | gef. 67,15 % C; 3,88 % H; 15,69 % N; 13,12 % Cl. |

### Beispiel 2: Herstellung von 2-Chlor-4,6-diphenyl-1,3,5-triazin

24,3 g (1,0 Mol) mit Iod aktivierte Magnesiumspäne in 40 ml wasserfreiem THF werden kurzzeitig bis auf ca. 60°C aufgeheizt. Darauf werden 113,1 g (1,0 Mol) Chlorbenzol und zusätzlich ca. 4 % Brombenzol sowie 220 ml THF innerhalb von 50 Min. zugetropft und anschließend für 2,5 Stunden auf Rückflußtemperatur gehalten. Nach Abkühlen wird die Grignard-Lösung innerhalb von 2 Stunden zu einer Mischung von 68,2 g (0,37 Mol) Cyanursäurechlorid und 215 ml THF gegeben, wobei die Temperatur der Mischung im Bereich von 0 bis 15°C gehalten wird. Nach beendeter Zugabe wird die Mischung bei 15°C gerührt, bis im Dünnschicht-Chromatogramm (Laufmittel Toluol:Hexan = 1:1) kein Monoaryltriazin mehr zu erkennen ist. Dies ist nach ca. 2,5 Stunden der Fall.
Anschließend wird die Mischung mit 300 ml Toluol verdünnt, die erhaltene Suspension auf 316 g 12 % wässrige HCl gegossen, die organische Phase abgetrennt, mit insgesamt 400 g Wasser gewaschen und eingeengt. Das erhaltene Material (Schmelzbereich 118-128°C) enthält 81 % der Titelverbindung. Die Ausbeute beträgt 81 %.

### Beispiel 3: Herstellung von 2-Chlor-4,6-di-(4-methylphenyl)-1,3,5-triazin

17,0 g (0,7 Mol) mit Iod aktivierte Magnesiumspäne in 40 ml wasserfreiem THF werden kurzzeitig bis auf ca. 60°C aufgeheizt. Darauf werden 122,2 g (0,7 Mol) 1-Brom-4-methylbenzol und 140 ml THF innerhalb von 30 Min. zugetropft und anschließend für 2 Stunden auf Rückflußtemperatur gehalten. Nach Abkühlen wird die Grignard-Lösung innerhalb von 1 Stunde zu einer Mischung von 43,0 g (0,23 Mol) Cyanursäurechlorid und 150 ml THF gegeben, wobei die Temperatur der Mischung im Bereich von 0 bis 10°C gehalten wird. Nach beendeter Zugabe wird die Mischung bei 0 bis 15°C gerührt, bis im Dünnschicht-Chromatogramm (Laufmittel Toluol:Hexan = 1:1) kein Monoaryltriazin mehr zu erkennen ist. Dies ist nach ca. 2,5 Stunden der Fall.

Anschließend wird die Mischung mit 210 ml Toluol verdünnt, die erhaltene Suspension auf 221 g 12 % wässrige HCl gegossen, die organische Phase abgetrennt, mit insgesamt 280 g Wasser gewaschen, eingeengt und mit 486 g 2-Butanol gereinigt. Das erhaltene Material (Schmelzpunkt 205-206°C) enthält 99 % der Titelverbindung. Die Ausbeute beträgt 73 %.

### Beispiel 4: Herstellung von 2-Chlor-4,6-di-(2,4-dimethylphenyl)-1,3,5-triazin

24,3 g (1,0 Mol) mit Iod aktivierte Magnesiumspäne in 40 ml wasserfreiem THF werden kurzzeitig bis auf ca. 60°C aufgeheizt. Darauf werden 221,2 g (1,0 Mol) 1-Brom-2,4-dimethylbenzol und 220 ml THF innerhalb von 60 Min. zugetropft und anschließend für 1,5 Stunden auf Rückflußtemperatur gehalten. Nach Abkühlen wird die Grignard-Lösung innerhalb von 1,5 Stunden zu einer Mischung von 61,5 g (0,33 Mol) Cyanursäurechlorid und 215 ml THF gegeben, wobei die Temperatur der Mischung im Bereich von 0 bis 5°C gehalten wird. Nach beendeter Zugabe wird die Mischung bei 5-25°C gerührt, bis im Dünnschicht-Chromatogramm (Laufmittel Toluol:Hexan = 1:1) kein Monoaryltriazin mehr zu erkennen ist. Dies ist nach 20 Stunden der Fall.
Anschließend wird die Mischung mit 300 ml Toluol verdünnt, die erhaltene Suspension auf 316 g 12 % wässrige HCl gegossen, die organische Phase abgetrennt, mit insgesamt 900 g Wasser gewaschen und eingeengt. Das erhaltene Material (Schmelzpunkt 135,5-137°C) enthält 97,3 % der Titelverbindung. Die Ausbeute beträgt 72 %.

### Beispiel 5: Herstellung von 2,4-Dichlor-6-phenyl-1,3,5-triazin

7,80 g (0,32 Mol) mit Iod aktivierte Magnesiumspäne in 20 ml wasserfreiem THF werden kurzzeitig bis auf 60°C aufgeheizt. Darauf werden 47,1 g (0,30 Mol) Brombenzol und 80 ml THF innerhalb von 30 Minuten zugetropft und für 1,5 Stunden auf 65°C erhitzt.
Nach Abkühlen wird die Grignard-Lösung innerhalb einer Stunde zu einer Mischung von 51,6 g (0,28 Mol) Cyanursäurechlorid und 140 ml THF gegeben, wobei die Temperatur der Mischung im Bereich von 10 bis 20°C gehalten wird. Nach beendeter Zugabe wird die Mischung für 3 Stunden bei 25°C gerührt. Anschließend wird die Mischung mit 280 ml Toluol verdünnt, die erhaltene Suspension auf 300 g 12 % wässrige HCl gegossen, die organische Phase abgetrennt, mit insgesamt 400 g Wasser gewaschen und eingeengt. Das erhaltene Material (Schmelzpunkt 107-116°C) wird aus Hexan umkristallisiert. Man erhält die Titelverbindung vom Schmelzpunkt 117-120°C in einer Ausbeute von 61 %.

### Beispiel 6: Herstellung von 2,4-Dichlor-6-(2,4-dimethylphenyl)-1,3,5-triazin

17,0 g (0,70 Mol) mit Iod aktivierte Magnesiumspäne in 25 ml wasserfreiem THF werden kurzzeitig bis auf 60°C aufgeheizt. Darauf werden 154,9 g (0,70 Mol) 4-Brom-m-Xylol (Reinheit ca. 90 %) und 155 ml THF innerhalb von 30 Minuten zugetropft und die Mischung für 2 Stunden zum Rückfluß erhitzt. Nach Abkühlen wird die Grignard-Lösung innerhalb von 1,5 Stunden zu einer Mischung von 43,0 g (0,233 Mol) Cyanursäurechlorid und 150 ml THF gegeben, wobei die Temperatur der Mischung im Bereich von 0 bis 10°C gehalten wird. Anschließend wird die Mischung mit 210 ml Toluol verdünnt, die erhaltene Suspension auf 220 g 12 % wässrige HCl gegossen, die organische Phase abgetrennt, mit insgesamt 280 g Wasser gewaschen und eingeengt. Das erhaltene Material wird aus 2-Butanol umkristallisiert. Man erhält die Titelverbindung vom Schmelzpunkt 135,5-137°C in einer Ausbeute von 79,8 %.

## Patentansprüche

1. Verfahren zur Herstellung einer Einzelverbindung der Formel Ia, 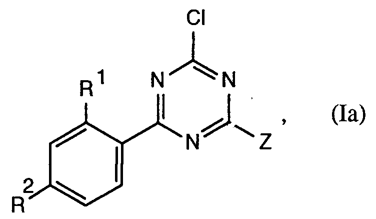 worin R¹ und R² unabhängig voneinander Wasserstoff oder C₁-C₄-Alkyl darstellen, und Z ein Rest der Formel Ib 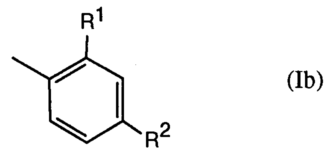 oder -Cl ist,
dadurch gekennzeichnet, daß man eine Verbindung der Formel II 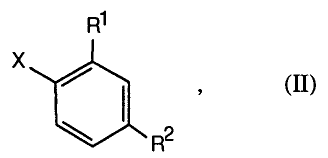 worin X für ein Brom- oder ein Chloratom steht und R¹ und R² die oben angegebenen Bedeutungen haben, in Tetrahydrofuran mit metallischem Magnesium zur entsprechenden Grignard-Verbindung reagieren läßt und die erhaltene Lösung mit Cyanursäurechlorid der Formel III 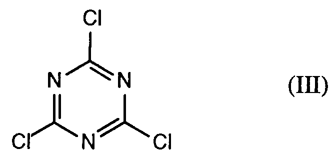 umsetzt, wobei auf 1 Mol Cyanursäurechlorid im Fall, daß Z -Cl ist, mindestens 1,05 Mol der Grignard-Verbindung eingesetzt werden, und im Fall, daß Z ein Rest der Formel Ib ist, mindestens 2,1 Mol der Grignard-Verbindung eingesetzt werden.

2. Verfahren gemäß Anspruch 1, worin R¹ die Bedeutung Wasserstoff oder Methyl hat und R² Wasserstoff oder C₁-C₄-Alkyl bedeutet.

3. Verfahren gemäß Anspruch 2, worin R¹ und R² unabhängig voneinander Wasserstoff oder Methyl bedeuten.

4. Verfahren gemäß Anspruch 1, worin die Verbindung der Formel II Chlorbenzol, Brombenzol, 4-Chlor-toluol, 4-Brom-toluol, 2,4-Dimethyl-brombenzol oder 2,4-Dimethyl-chlorbenzol ist.

5. Verfahren gemäß Anspruch 1, worin die weitere Aufreinigung der Verbindung der Formel I ohne Destillation vorgenommen wird.

6. Verfahren gemäß Anspruch 1, worin außer Tetrahydrofuran kein weiteres Lösungsmittel eingesetzt wird.

7. Verfahren gemäß Anspruch 1, worin für die Umsetzung mit Cyanursäurechlorid neben Tetrahydrofuran ein zusätzliches Lösungsmittel eingesetzt wird.

8. Verfahren gemäß Anspruch 7, worin als zusätzliches Lösungsmittel ein weiterer Ether oder ein aliphatischer oder aromatischer Kohlenwasserstoff oder ein Gemisch dieser Lösungsmittel eingesetzt wird.

9. Verfahren gemäß Anspruch 8, worin als zusätzliches Lösungsmittel Dioxan, Toluol, Xylol, Hexan, Cyclohexan, Petrolether oder ein Gemisch dieser Lösungsmittel eingesetzt wird.

10. Verfahren gemäß Anspruch 7, worin die Lösung der Grignard-Verbindung in Tetrahydrofuran einer Lösung von Cyanursäurechlorid in dem zusätzlichen Lösungsmittel zugegeben wird.

11. Verfahren gemäß Anspruch 1, worin auf ein Mol Cyanursäurechlorid im Fall, daß Z -Cl ist, 1,1 bis 2 Mole der Grignard-Verbindung eingesetzt werden, und im Fall, daß Z ein Rest der Formel Ib ist, 2,2 bis 4 Mole der Grignard-Verbindung eingesetzt werden.

12. Verfahren gemäß Anspruch 11, worin auf ein Mol Cyanursäurechlorid im Fall, daß Z -Cl ist, 1,25 bis 1,5 Mole der Grignard-Verbindung eingesetzt werden, und im Fall, daß Z ein Rest der Formel Ib ist, 2,5 bis 3 Mol Grignard-Verbindung eingesetzt werden.

13. Verfahren gemäß Anspruch 1, worin Cyanursäurechlorid als Lösung in Tetrahydrofuran, Toluol oder einer Mischung aus Toluol und Tetrahydrofuran vorgelegt und dazu die Grignard-Verbindung als Lösung in Tetrahydrofuran zugegeben wird.

14. Verfahren gemäß Anspruch 1, worin die Reaktionsmischung während der Umsetzung mit dem Cyanursäurechlorid im Temperaturbereich von -5°C bis +45°C gehalten wird.

15. Verfahren gemäß Anspruch 1 zur Herstellung von 2-Chlor-4,6-diaryl-1,3,5-triazinen der Formel I, 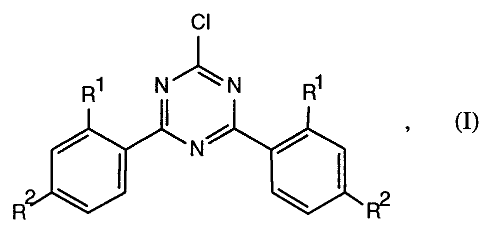 worin R¹ und R² unabhängig voneinander Wasserstoff oder C₁-C₄-Alkyl darstellen, dadurch gekennzeichnet, daß man eine Verbindung der Formel II 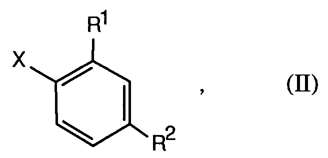 worin X für ein Brom- oder ein Chloratom steht und R¹ und R² die oben angegebenen Bedeutungen haben, in Tetrahydrofuran mit metallischem Magnesium zur entsprechenden Grignard-Verbindung reagieren läßt und die erhaltene Lösung mit Cyanursäurechlorid der Formel III 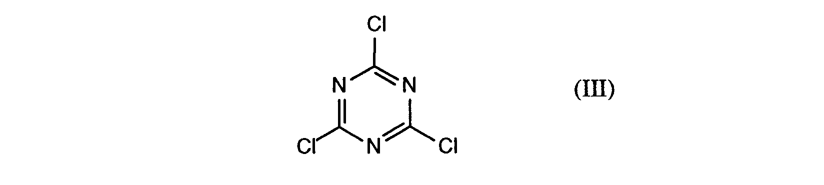 umsetzt, wobei auf 1 Mol Cyanursäurechlorid mindestens 2,1 Mol der Grignard-Verbindung eingesetzt werden.
